**Europäisches Patentamt**

**European Patent Office**

**Office européen des brevets**

(19)

(11) Veröffentlichungsnummer: **0 158 712**
**B1**

(12) # EUROPÄISCHE PATENTSCHRIFT

(45) Veröffentlichungstag der Patentschrift:
24.05.89

(51) Int. Cl.⁴: **G 05 F 1/12,** A 61 N 1/40

(21) Anmeldenummer: 84113826.6

(22) Anmeldetag: 15.11.84

(54) **Regeleinrichtung für Kurzwellentherapiegeräte.**

(30) Priorität: 22.03.84 DD 261118

(43) Veröffentlichungstag der Anmeldung:
23.10.85 Patentblatt 85/43

(45) Bekanntmachung des Hinweises auf die Patenterteilung:
24.05.89 Patentblatt 89/21

(84) Benannte Vertragsstaaten:
AT DE NL SE

(56) Entgegenhaltungen:
DD-A- 2 494
DD-A- 15 829
US-A- 4 230 129
US-A- 4 285 346

Buch von Hilberg/Piloty, "Mikroprozessoren und ihre
Anwendungen" (1977) Verlag R. Oldenbourg, Bd. 1,
Seiten 44 - 49 und 281

(73) Patentinhaber: VEB Transformatoren- und Röntgenwerk
"Hermann Matern", Overbeckstrasse 48,
DDR-8030 Dresden (DD)

(72) Erfinder: Igel, Gerald, Dipl.-Ing.,
Karl-Liebknecht-Strasse 13,
DDR-1406 Hohen-Neuendorf (DD)

(74) Vertreter: Patentanwälte Beetz sen. - Beetz jun. Timpe -
Siegfried - Schmitt-Fumian, Steinsdorfstrasse 10,
D-8000 München 22 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf eine Regeleinrichtung für Kurzwellentherapiegeräte mit automatischer Leistungsanpassung durch Drehrichtungssteuerung eines Abstimmotors, der eine Impedanzanpassung des Behandlungskreises an den Sender vornimmt.

Bei Kurzwellentherapiegeräten ist der Patient als Verbraucher an einen Hochfrequenzschwingkreis angekoppelt. Dabei wird die HF-Leistung in einer selbsterregten HF-Generatorstufe erzeugt, über Filter geführt und mittels eines primären Schwingkreises schließlich dem Patientenkreis zugeführt. Damit eine maximale Leistungsübertragung erreicht wird, ist es notwendig, daß der Patientenkreis an den Schwingkreis angepaßt ist. Durch Patientenbewegungen treten jedoch Impendanzänderungen im Behandlungskreis und damit Änderungen der jeweils applizierten Leistung ein. Deshalb ist es üblich, die Impedanzänderung im Behandlungskreis durch eine Kapazitätsänderung auszugleichen. Die Kapazitätsänderung erfolgt durch einen im Behandlungskreis liegenden Drehkondensator, dessen Achse mit einem in seiner Drehrichtung umsteuerbaren Motor mechanisch fest gekoppelt ist (DD-A1 15 829 und DD-A1 24 94. Zur Steuerung des Abstimmotors ist es ferner bekannt, eine Schaltungsanordnung vorzusehen, bei der ein der Hochfrequenzleistung proportionales Signal über ein Filter zu einer Schaltstufe gelangt, in der es im 100-Hz-Rhythmus auf einen Speicherkondensator gegeben und gleichzeitig der 0,95-fache Spitzenwert über einen Spannungsteiler gebildet und gespeichert wird. Beide Spannungswerte gelangen an die Eingänge eines Komparators, dessen Ausgangspegel je nach Größe bzw. Polarität der beiden Spannungen zueinander sprunghaft wechselt. Durch eine am Komparatorausgang angeschlossene Impulsformerstufe werden Rechteckimpulse gebildet, mit denen einerseits die Entladung des 0,95-fachen Spitzenwertes vorgenommen und andererseits ein T-Flipflop gesteuert wird. Dieses steuert dann letztlich über zwei Triacs jeweils eine Wicklung des Abstimmotors für den Drehkondensator.

Bei Anwendung neuer Behandlungsverfahren in der Kurzwellentherapie wird jedoch in zunehmendem Maße mit Impulskurzwellen gearbeitet. In diesem Fall wird die Hochfrequenz nur in kurzen Impulsgruppen konstanter HF-Leistung und konstanter Impulsdauer (z. B. 400 µs) dem Patienten zugeführt. Eine Leistungsregelung erfolgt durch eine Veränderung der Impulsfolgefrequenz, die etwa 20 bis 200 Hz beträgt.

Die eingangs beschriebene herkömmliche Schaltungsanordnung ist für die automatische Leistungsanpassung bei Impulsbetrieb nicht mehr verwendbar, da bereits durch das Tiefpaßfilter kein proportionaler Leistungswert mehr zur Verfügung steht. Auch eine Meßwertabtastung im Rhythmus von 100 Hz müßte bei 400 µs Impulsdauer auf 2,5 kHz synchronisiert werden. Des weiteren ist die Totzeit der folgenden Baugruppen zu groß, so daß eine solche Abstimmautomatik nicht mehr auf das Maximum der HF-Leistung abstimmen, sondern auf die Tastimpulse der HF-Leistung ansprechen würde.

Es ist Aufgabe der Erfindung, den vorstehend beschriebenen Mangel zu beseitigen und eine Regeleinrichtung für Kurzwellentherapiegeräte anzugeben, die infolge ihrer geringen Totzeit sowohl in Geräten für Dauerbetrieb als auch in Geräten für Impulsbetrieb verwendet werden kann. Die Aufgabe wird gemäss Patentanspruch 1 gelöst. Vorteilhafte Weiterbildungen der Erfindungskonzeption sind Gegenstand der abhängigen Ansprüche.

Die erfindungsgemäße Regeleinrichtung für Kurzwellentherapiegeräte mit automatischer Leistungsanpassung durch Kapazitätsänderung eines Drehkondensators über einen Abstimmotor mit umsteuerbarer Drehrichtung unter Anpassung des HF-Senders an den den Patienten einschließenden Behandlungskreis ist gekennzeichnet durch

A) einen Mikrocomputer mit
   a1) einer zentralen Verarbeitungseinheit (CPU),
   a2) einem Festwertspeicher (ROM) für Instruktionen für die Programmierung eines Ein- und Ausgabeschaltkreises (PIO) sowie für die Koordinierung des Datenverkehrs zwischen den zugehörigen Baugruppen und
   a3) einem Schreib-Lese-Speicher (RAM) für die Speicherung von Zwischenergebnissen,

B) einen Analog-Digital-Umsetzer, der die abgegebene HF-Leistung repräsentierende Signale in entsprechende Digitalsignale umsetzt und sie an die zentrale Verarbeitungseinheit CPU liefert,

C) einen Ein- und Ausgabeschaltkreis (PIO), der
   c1) Veränderungen bei einstellbaren Parametern durch die Bedienungsperson der (CPU) übermittelt,
   c2) die Drehrichtung für den Abstimmotor steuert
und
   c3) die Spannungsversorgung des HF-Senders entsprechend der voreingestellten Leistungsstufe freigibt,
und

(D) einen Zeitgeberschaltkreis (CTC), der als von der zentralen Verarbeitungseinheit (CPU) gesteuerter Frequenzteiler arbeitet, zur Erzeugung einer Tastfrequenz für die Tastung des HF-Senders bei Impulsbetrieb und/oder eine netzsynchrone Frequenzquelle für die Tastung des HF-Senders bei Dauerbetrieb.

Die mit der Erfindung erreichten Vorteile bestehen vor allem darin, daß auch bei impulsartiger Beaufschlagung des Patienten mit HF-Energie stets die eingestellten Leistungswerte auch tatsächlich erreicht und somit reproduzierbare Bedingungen eingehalten werden.

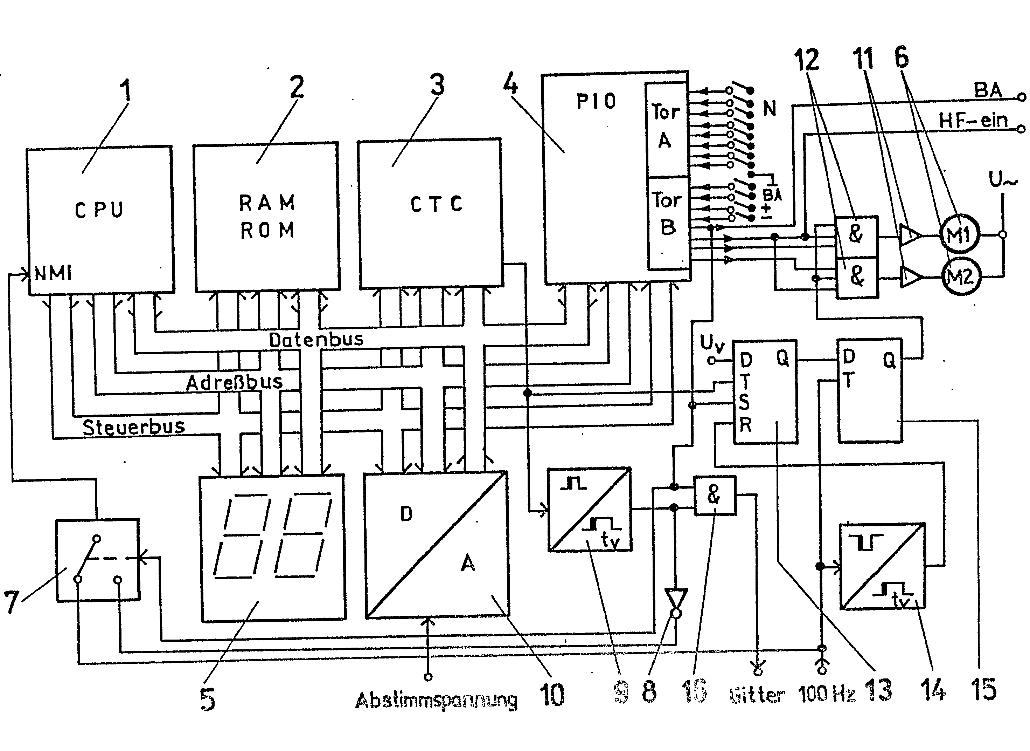

Die Erfindung wird nachfolgend anhand eines Ausführungsbeispiels und unter Bezugnahme auf die Zeichnung näher erläutert.

Die Zeichnung zeigt ein schematisches Blockschaltbild der erfindungsgemäßen Regeleinrichtung für Kurzwellentherapiegeräte. Sie weist einen Mikrocomputer auf, der die gesamten Regel- und Steuerfunktionen des Geräts übernimmt. Der Mikrocomputer (bzw. Mikroprozessor) weist wie üblich eine zentrale Verarbeitungseinheit (CPU) 1, einen Festwertspeicher (ROM) mit Instruktionen für die CPU 1 sowie einen Schreib-Lese-Speicher (RAM) auf. In der Zeichnung gilt für beide Speicher (ROM und RAM) die Bezugszahl 2. Aufgabe der Speicher 2 ist das Einschreiben und Auslesen der variablen Daten für den Betrieb der CPU 1. Zusätzlich ist eine Zeitgeberschaltung (CTC) 3 vorgesehen, die sowohl die Funktion eines Frequenzteilers als auch die eines durch die CPU 1 steuerbaren Frequenzgenerators übernimmt. Zur Kommunikation des Mikrocomputers mit seiner Peripherie und seiner Beeinflussung durch die Bedienungsperson besitzt der Mikrocomputer mehrere Ein- und Ausgabeschaltkreise (PIO) 4. Wenn der Mikrocomputer durch Anlegen der Versorgungsspannung in Betrieb gesetzt wird, führt die CPU 1 eine Leseoperation an der Adresse Null des ROM's aus. Damit wird der CPU 1 der Code für den ersten auszuführenden Befehl übermittelt. Nach Abarbeitung dieses Befehls wird wieder eine Leseoperation im ROM 2 durchgeführt und damit der Code für den nächsten Befehl ausgelesen. Dementsprechend werden die Betriebsinstruktionen der Reihe nach ausgelesen und ausgeführt. Nach einer Programmierung aller Schaltkreise (PIO 4 und CTC 3) sowie dem Rücksetzen einer vorhandenen digitalen Anzeigeeinheit 5 für die Behandlungszeit wird kontrolliert, ob sich alle Tasten des Kurzwellentherapiegerätes in einem Zustand entsprechend der Bedienungsanweisung befinden. Ist dies nicht der Fall, nimmt das Steuerungssystem so lange einen Wartezustand ein, bis die Tastenstellung dem geforderten Zustand entspricht. Befindet sich die Tastatur nicht in dem gewünschten Zustand, so wird dies durch ein akustisches Signal angezeigt. Entspricht die Tastenstellung dem geforderten Zustand, dann beginnt die ständige Tastenabfrage. Die Behandlungszeit läßt sich mittels einer Zweitastenbedienung durch Auf- bzw. Abwärtszählung verändern. Die dafür notwendigen Tasten «+», «−» sind am PIO 4 angeschaltet. Die aktuelle Behandlungszeit kann an der beispielsweise als Siebensegmentanzeige ausgeführten Anzeigeeinheit 5 abgelesen werden. Ein Betriebsartenwechsel zwischen Dauerbetrieb und Impulsbetrieb ist durch einmaliges Drücken der Taste BA möglich.

Im folgenden wird die Arbeitsweise der erfindungsgemäßen mikrocomputergesteuerten Regeleinrichtung näher erläutert. Nach Betätigen einer der Leistungsbereichstasten N des PIO 4 wird die Spannungsversorgung einer in der Zeichnung nicht dargestellten Senderöhre eingeschaltet. Gleichzeitig wird eine Wicklung des Abstimmotors 6 über Verstärker 11 und UND-Glieder 12 angesteuert, so daß der Abstimmotor 6 die Impedanz des Ausgangskreises an die jeweilige Patientenimpedanz anpaßt. Der Spannungsabfall an einem in der Spannungsversorgung des nicht dargestellten Frequenzgenerators angeordneten Widerstand ist dabei ein Maß für die Anpassung des Behandlungskreises an den Patienten. In Abhängigkeit von der an einem Ausgang BA des PIO 4 eingestellten Betriebsart wird entweder bei Dauerbetrieb netzsynchron oder bei Impulsbetrieb in der Mitte des Freigabeimpulses für die Senderöhre dieser Spannungsabfall abgetastet. Dieses Abtastprogramm besitzt auf der Grundlage der Regelfunktion der im Patienten umgesetzten Leistung die höchste Priorität. Aus diesem Grunde erfolgt die Auslösung dieses Programms durch einen nicht maskierbaren Interrupt (NMI). Dementsprechend wird der Eingang NMI der CPU 1 in einem NMI-Schalter 7 in Abhängigkeit vom Ausgang BA des PIO 4 entweder mit einem netzsynchronen Signal oder über einen Negator 8 mit dem Ausgang eines monostabilen Multivibrators 9, der die Auftastzeit der nicht gezeigten HF-Senderöhre bestimmt, verbunden.

Ein Analog-Digital-Umsetzer (A/D-Umsetzer) 10 digitalisiert ständig die an seinem Eingang liegende Abstimmspannung. Zum Zeitpunkt der Abtastung legt er auf Anforderung durch die CPU 1 den Digitalwert der Abstimmspannung auf den Datenbus. Die CPU 1 schreibt diesen Wert in ihren Akkumulator. In der CPU 1 wird jeder neue Digitalwert mit dem vorhergehenden abgespeicherten Wert verglichen. Wenn der aktuelle Digitalwert größer ist als der vorhergehende, wird der neue Wert eingespeichert. Ist er dagegen kleiner als der vorhergehende Wert, wird er mit dem 0,95-fachen vorherigen Wert verglichen. Nur wenn das Ergebnis auch kleiner als dieser Wert ist, erfolgt die Drehrichtungsänderung des Abstimmotors 6 über die Wicklungen M1 bzw. M2. Während der Zeitspanne, in der keine Abfrage der Abstimmspannung über den A/D-Umsetzer 10 erfolgt, fragt der Mikrocomputer in seinem Steuerungssystem alle Schaltzustände der Tasten ab und stellt fest, ob eine neue Funktion ausgelöst werden muß, z.B. bei Ablauf der Behandlungszeit, Änderung der Leistung oder des Tastverhältnisses, oder ob eine Notabschaltung durch den Patienten erfolgte.

Bei der Wahl der Betriebsart «Impulsbetrieb» ist folgendes von Bedeutung: Bei einer Impulsfolgefrequenz von 20 Hz bis 200 Hz und einer geforderten Pendelfrequenz des Abstimmotors von 3 Hz bis 5 Hz, was einem Leistungsabfall von ca. 5% gegenüber maximal möglicher Leistung entspricht, würden zwischen zwei Umschaltpunkten bei den niedrigen Impulsfolgefrequenzen nur ungefähr vier Meßwerterfassungen erfolgen. Um in diesen Fällen eine ausreichende Anzahl von Meßwerterfassungen und damit eine hohe Störsicherheit zu gewährleisten, wird der Abstimmotor 6 so angesteuert, daß er nach jedem Impuls nur für die Dauer einer Halbwelle der Netzspannung in Betrieb ist, also für 10 ms. Die prinzipielle Wirkungsweise dieses Steuerungssystems wird nachfolgend beschrieben. Am PIO 4 liegt wiederum das dem Betriebszustand «Impulsbetrieb» entsprechende Signal. An den Ausgang des CTC 3 ist der Takteingang T eines D-Flipflops 13 angeschaltet.

Damit wird sein Ausgang Q bei jedem Impuls auf den Logikpegel H gesetzt. Zu Beginn jeder Halbwelle wird der Rücksetzeingang R über eine monostabile Kippstufe 14 angesteuert und damit der Ausgang Q auf den Logikpegel L gebracht. Der Ausgang Q ist mit einem Eingang D eines weiteren D-Flipflops 15 verbunden, dessen Takteingang T das Abtastsignal, das 50 Hz oder 100 Hz betragen kann, zugeführt wird. Damit wird zu Beginn jeder Halbwelle der Zustand des Ausgangs Q des D-Flipflops 13 auf den Ausgang Q des D-Flipflops 15 übertragen und somit der Abstimmotor 6 für jeweils eine Halbwelle angesteuert. Damit beim Betriebszustand «Dauerbetrieb» der Abstimmotor ständig angeschaltet bleibt, ist der Setzeingang S des D-Flipflops 13 mit dem Ausgang BA des PIO 4 verbunden. Damit in dieser Betriebsart das Gitter der nicht gezeigten HF-Senderöhre ständig freigegeben ist, ist der Ausgang des monostabilen Multivibrators 9 mit dem Eingang einer UND-Schaltung 16 verbunden, an deren zweiten Eingang der Ausgang BA des PIO 4 und an deren Ausgang das Gitter der Senderöhre angeschaltet sind. Der CTC 3, der die Taktfrequenz der CPU 1 teilt und einerseits mit der so erzeugten und programmierbaren Frequenz die Senderöhre im Impulsbetrieb tastet, wird andererseits auch gleichzeitig dazu herangezogen, die Behandlungszeit zu messen. Dies erfolgt dadurch, daß von der im Register der CPU 1 bzw. des RAM 2 eingeschriebenen und in der Anzeigeeinheit 5 angezeigten geplanten Behandlungszeit im Minutentakt durch die CPU 1 die tatsächliche durch die CTC 3 bestimmte Behandlungszeit subtrahiert wird, so daß der Anzeigeeinheit stets die noch verbleibende Behandlungszeit zur Anzeige kommt.

**Patentansprüche**

1. Regeleinrichtung für Kurzwellentherapiegeräte mit automatischer Leistungsanpassung durch Kapazitätsänderung eines Drehkondensators über einen Abstimmotor mit umsteuerbarer Drehrichtung unter Anpassung des HF-Senders an den den Patienten einschließenden Behandlungskreis, gekennzeichnet durch

A) einen-Mikrocomputer mit
   a1) einer zentralen Verarbeitungseinheit (CPU) (1),
   a2) einem Lesespeicher (ROM) (2) für Instruktionen für die Programmierung eines Ein- und Ausgabeschaltkreises (PIO) (4) sowie für die Koordinierung des Datenverkehrs zwischen den zugehörigen Baugruppen und
   a3) einem Schreib-Lese-Speicher (RAM) (2) für die Speicherung von Zwischenergebnissen,

B) einen Analog-Digital-Umsetzer (10), der die abgegebene HF-Leistung repräsentierende Signale in entsprechende Digitalsignale umsetzt und sie an die zentrale Verarbeitungseinheit CPU (1) liefert,

C) einen Ein- und Ausgabeschaltkreis (PIO) (4), der

c1) Veränderungen bei einstellbaren Parametern (N) durch die Bedienungsperson der (CPU) (1) übermittelt,
c2) die Drehrichtung für den Abstimmotor (6) steuert
und
c3) die Spannungsversorgung des HF-Senders entsprechend der voreingestellten Leistungsstufe (N) freigibt,
und

(D) einen Zeitgeberschaltkreis (CTC) (3), der als von der zentralen Verarbeitungseinheit (CPU) (1) gesteuerter Frequenzteiler arbeitet, zur Erzeugung einer Tastfrequenz für die Tastung des HF-Senders bei Impulsbetrieb und/ oder eine netzsynchrone Frequenzquelle für die Tastung des HF-Senders bei Dauerbetrieb.

2. Regeleinrichtung nach Anspruch 1, dadurch gekennzeichnet, daß der Ausgabebefehl des Zeitgeberschaltkreises (CTC) (3) die Einschaltung der Kurzwellenleistung über einen monostabilen Multivibrator (9) bewirkt, dessen Haltezeit die Auftastzeit des Hochfrequenzimpulses der Senderöhre bestimmt.

3. Regeleinrichtung nach Anspruch 1 oder 2, dadurch gekennzeichnet, daß der Ausgang des monostabilen Multivibrators (9) mit einem Eingang einer UND-Schaltung (16) verbunden ist, deren anderer Eingang mit einem Betriebsartensignal, das zwischen Dauer- und Impulsbetrieb unterscheidet, in Verbindung steht und deren Ausgang das Gitter der Senderöhre steuert.

4. Regeleinrichtung nach einem der Ansprüche 1 bis 3, dadurch gekennzeichnet, daß die zentrale Verarbeitungseinheit (CPU) (1) einen Eingang für nicht maskierbaren Interrupt (NMI) besitzt, der in Abhängigkeit von der Betriebsart mit dem Ausgang des monostabilen Multivibrators (9) oder mit der netzsynchronen Frequenzquelle verbunden ist.

5. Regeleinrichtung nach einem der Ansprüche 1 bis 4, dadurch gekennzeichnet, daß in der zentralen Verarbeitungseinheit (CPU) (1) oder im Schreib-Lese-Speicher (RAM) (2) ein Register für die Speicherung der gewünschten Behandlungszeit, die über den Ein- und Ausgabeschaltkreis (PIO) (4) einstellbar ist, sowie für eine digitale Zeitanzeige der Behandlungszeit vorhanden ist, die in durch den Zeitgeberschaltkreis (CTC) (3) erzeugtem Minutentakt durch die zentrale Verarbeitungseinheit (CPU) (1) in Einerschritten verringert wird, und daß die zentrale Verarbeitungseinheit (CPU) (1) nach Erreichen des Wertes Null in diesem Register die Hochfrequenzleistungsabschaltung auslöst.

6. Regeleinrichtung nach einem der Ansprüche 1 bis 5, dadurch gekennzeichnet, daß zur Halbwellensteuerung des Abstimmotors (6) ein D-Flipflop (13) vorgesehen ist, dessen Takteingang (T) mit dem Ausgang des Zeitgeberschaltkreises (CTC) (3) verbunden ist und dessen Rücksetzeingang (R) am Ausgang einer als Verzögerungsschaltung arbeitenden monostabilen Kippstufe (14) anliegt, ein Ausgang (Q) des D-Flipflops (13) mit einem Eingang (D) eines weiteren D-Flipflops (15) verbunden ist, dessen Ausgang (Q) mit jeweils einem Eingang zweier UND-Glieder (12) in Verbindung steht, und die Ausgänge der UND-Glieder (12) über elektronische Verstärker (11) mit den Wicklungen ($M_1$, $M_2$) des Abstimmotors (6) verbunden sind.

7. Regeleinrichtung nach einem der Ansprüche 1 bis 6, dadurch gekennzeichnet, daß der Zeitgeberschaltkreis (CTC) (3) zur Steuerung einer Behandlungsuhr dient.

## Claims

1. Control device for short-wave therapy apparatus including automatic power adjustment by variation of the capacity of a turning condenser via a tuning motor with reversible direction of rotation, adjusting the high frequency transmitter to the treatment circuit including the patient, characterized by

A) a micro-computer with
   a1) a central processing unit (CPU) (1),
   a2) a read only memory (ROM) (2) for instructions to program an input and output circuit (PIO) (4) and to coordinate the data exchange between the associated assemblies, and
   a3) a write-read memory (RAM) (2) for the storage of intermediate results,

B) an analog-digital converter (10) for converting the signals representing the HF power output into corresponding digital signals and for supplying them to a central processing unit CPU (1).

C) an input and output circuit (PIO) (4) for
   c1) transmitting variations of parameters (N) adjustable by the operator to the (CPU) (1),
   c2) controlling the direction of rotation for the tuning motor (6), and for
   c3) releasing the voltage supply of the HF transmitter in accordance with the preset power stage (N), and

D) a timing circuit (CTC) (3) operative as a frequency divider controlled by the central processing unit (CPU) (1) for the generation of a sampling frequency for sampling the HF transmitter during pulse operation and/or a net synchronous frequency source for the sampling of the HF transmitter during continuous operation.

2. The control device according to claim 1, characterized in that the output instruction of the timing circuit (CTC) (3) causes switching on of the short-wave power via a monostable multivibrator (9) whose hold time determines the sampling time of the high frequency pulse of the transmitter tube.

3. The control device according to claim 1 or claim 2, characterized in that the output of the monostable multivibrator (9) is connected to one input of an AND circuit (16) whose other input communicates with an operating mode signal differentiating between continuous operation and pulse operation and whose output controls the grid of the transmitter tube.

4. The control device according to one of claims 1 to 3, characterized in that the central processing unit (CPU) (1) has an input fot non-maskable interrupt (NMI) which, depending on the mode of operation, is connected to the output of the monostable multivibrator (9) or to the net synchronous frequency source.

5. The control device according to one of claims 1 to 4, characterized in that in the central processing unit (CPU) (1) or in the write-read memory (RAM) (2) a register for the storage of the desired duration of treatment, which may be set via the input and output circuit (PIO) (4), and for a digital time display of the duration of treatment is provided which is reduced in units steps by the central processing unit (CPU) (1) at a cycle time of one minute generated by the timing circuit (CTC) (3), and in that, after reaching the value zero, the central processing unit (CPU) (1) triggers high frequency power switch-off.

6. The control device according to one of claims 1 to 5, characterized in that for the halv-wave control of the tuning motor (6) a D-flip-flop (13) is provided whose pulse input (T) is connected to the output of the timing circuit (CTC) (3) and whose reset input (R) is connected to the output of a monostable flipping circuit (14), an output (Q) of the D-flip-flop (13) is connected to an input (D) of a further D-flip-flop (15) whose output (Q) is connected to an input of two AND elements (12), respectively, and in that the outputs of the AND elements (12) are connected to the windings ($M_1$, $M_2$) of the tuning motor (6) via electronic amplifiers (11).

7. The control device according to one of claims 1 to 6, characterized in that the timing circuit (CTC) (3) serves to control a treatment clock.

## Revendications

1. Dispositif de régulation pour des appareils de thérapie par ondes courtes avec une adaptation automatique de la puissance par modification de la capacité d'un condensateur tournant, par l'intermédiaire d'un moteur de réglage dont le sens de rotation est susceptible d'être inversé et en adaptant l'émetteur HF au circuit de traitement incluant les patients, dispositif de régulation caractérisé en ce qu'il comporte:

A) un microcalculateur avec:
   a1) une unité centrale de traitement (CPU) (1),
   a2) une mémoire morte (ROM) (2) pour des instructions destinées à la programmation d'un circuit de commutation entrée et sortie (PIO) (4) ainsi qu'à la coordination des échanges de données entre les groupes constitutifs faisant partie du dispositif,
   a3) une mémoire à lecture écriture (RAM) (2) pour la mémorisation de résultats intermédiaires,

B) Un convertisseur analogique-numérique (10) qui convertit les signaux représentatifs de la puissance HF délivrée en signaux numériques correspondants et qui les délivre à l'unité centrale de traitement CPU (1),

C) Un circuit de commutation entrée et sortie (PIO) (4) qui:

c1) transmet à l'unité centrale de traitement (CPU) (1) des modifications de paramètres réglables (N) par le personnel de service,

c2) commande le sens de rotation du moteur de réglage (6),

c3) libère l'alimentation en tension de l'émetteur HF de façon correspondante à l'échelon de puissance (N) préréglé,

D) Un circuit de commutation d'horloge (CTC) (3) qui fonctionne comme un diviseur de fréquence commandé par l'unité centrale de taitement (CPU) (1), pour engendrer une fréquence d'impulsion pour la modulation de l'émetteur HF en fonctionnement impulsionnel et/ou une source de fréquence en synchronisme avec le réseau pour la modulation de l'émetteur HF en fonctionnement permanent.

2. Dispositif de régulation selon la revendication 1, caractérisé en ce que l'ordre se sortie du circuit de commutation d'horloge (CTC) (3) provoque le déclenchement de la puissance en onde courte par l'intermédiaire d'un multivibrateur monostable (9) dont la période de maintien détermine la période de déclenchement de l'impulsion haute fréquence des tubes émetteurs.

3. Dispositif de régulation selon la revendication 1 ou 2, caractérisé en ce que la sortie du multivibrateur monostable (9) est reliée à une entrée d'un circuit ET (16) dont l'autre entrée est en liaison avec un signal de mode de fonctionnement qui établit la discrimination entre le fonctionnement permanent et le fonctionnement impulsionnel et dont la sortie commande la grille des tubes émetteurs.

4. Dispositif de régulation selon une des revendications 1 à 3, caractérisé en ce que l'unité centrale de traitement (CPU) (1) comporte une entrée pour une interruption non susceptible d'être masquée (NMI) qui, en fonction du mode de fonctionnement, est reliée à la sortie du multivibrateur monostable (9) ou bien à la source de fréquence en synchronisme avec le réseau.

5. Dispositif de régulation selon une des revendications 1 à 4, caractérisé en ce qu'il est prévu, dans l'unité centrale de traitement (CPU) (1) ou bien dans la mémoire à lecture-écriture (RAM) (2), un registre pour mémoriser le temps de traitement souhaité, lequel est réglable par l'intermédiaire du circuit d'entrée et sortie (PIO) (4), ainsi que pour afficher numériquement le temps de traitement qui est réduit étape par étape par l'unité centrale de traitement (CPU) (1) selon la cadence engendrée par le circuit de commutation d'horloge (CTC) (3), tandis que l'unité centrale de traitement (CPU) (1) après que la valeur zéro ait été atteinte dans ce rigstre. déclenche la coupure de la puissance haute fréquence.

6. Dispositif de régulation selon une des revendications 1 à 5, caractérisé en ce que pour la commande en demi-onde du moteur de réglage (6), il est prévu une bascule D (13) dont l'entrée de synchronisation (T) est reliée à la sortie du circuit de commutation d'horloge (CTC) (3), et dont l'entrée de remise à l'état initial (R) est appliquée à la sortie d'un étage basculant monostable (14) jouant le rôle de circuit de temporisation, une sortie (Q) de la bascule D (13) étant reliée à une entrée (D) d'une autre bascule D (15), dont la sortie (Q) est en liaison avec, respectivement une entrée de deux organes ET (12), tandis que les sorties de ces organes ET (12) sont reliées par l'intermédiaire d'amplificateurs électroniques (11) aux enroulements ($M_1$, $M_2$) du moteur de réglage 6.

7. Dispositif de régulation selon une des revendications 1 à 6, caractérisé en ce que le circuit de commutation d'horloge (CTC) (3) sert à commander une horloge de traitement.

7